# EUROPEAN PATENT APPLICATION

(11) **EP 0 938 904 A1**
(43) Date of publication of application: **01.09.1999**
(21) Application number: 98200382.4
(22) Date of filing: 09.02.1998
(51) Int. Cl.: A61K 48/00, C12N 5/10, A61K 35/28

(54) **Mammalian cell transduction for use in gene therapy**

(71) Applicant: Leuven Research & Development V.Z.W., 3000 Leuven (BE)
(72) Inventor: The designation of the inventor has not yet been filed
(74) Representative: Van Someren, Petronella F. H. M.

(57) **Abstract**

The present invention relates to a method for the ex vivo transduction of mammalian cells, in particular to the transduction of bone marrow stromal cells. These cells can be transduced with a gene of interest, in particular a B-domain deleted human factor VIII gene. In the latter case, the trasnduced cells can be used to treat hemophilia A. The method for the ex vivo transduction of bone marrow stromal cells with the human factor VIII gene comprises provision of an intron-based retroviral vector comprising a B-domain deleted human factor VIII cDNA (designated as MFG-FVIIIΔB);
pseudotyping the said vector with the Gibbon ape leukemia virus (GALV) envelope; transducing bone marrow stromal cells with the said pseudotyped vector by pre-incubating the cells for a suitable period of time in cell culture medium without phosphate and subsequently adding a vector-containing supernatant, optionally supplemented with transduction additives to the cells, followed by centrifuging the mixture thus obtained; and optionally repeating the two previous steps.

## Description

The present invention relates to a new method for the ex vivo transduction of mammalian cells. The invention further relates to genetically engineered cells thus obtained and to the use of these cells in gene therapy. The invention in particular relates to the transduction of human bone marrow stromal cells with a vector expressing a blood coagulation factor, in particular factor VIII.

Somatic gene therapy involves the genetic engineering of somatic cells and the administration of these cells to a subject in need of therapy. Through genetically engineering the cells, they will acquire one or more desirable properties they did not or did no longer possess, for example the ability to express a particular protein. As an alternative a cell may be genetically engineered to loose an unwanted property.

The first important step in genetically engineering cells for use in gene therapy is being capable of efficiently transducing the cells with a vector harboring the gene of interest and to obtain stable expression of that gene in the cells.

The present invention has for its general object to provide such a method of efficiently transducing cells ex vivo.

It is a more specific object of the present invention to provide such a method for obtaining cells for treating hemophilia A by means of gene therapy.

Hemophilia A is a congenital X-chromosome-linked coagulation disorder characterized by uncontrolled crippling hemorrhagic episodes which occurs in approximately 1/10000 males. Hemophilia A is due to a deficiency of coagulation factor VIII (FVIII), which accelerates the activation of factor X by activated factor IX in the presence of calcium and phospholipids. Ultimately the coagulation cascade leads to the localized generation of thrombin and the conversion of fibrinogen to insoluble fibrin polymers, which in conjunction with platelet aggregation maintains hemostasis.

Hemophilia A is particularly suitable for gene therapy since expression of FVIII does not require precise metabolic regulation and since a slight increase in plasma FVIII levels can potentially convert severe [FVIII: 1-2 ng/ml] to mild hemophilia [FVIII: 2-60 ng/ml]. Gene therapy for hemophilia A should provide constant, sustained synthesis within the patient, thereby obviating the risk of spontaneous bleeding, the need for repeated FVIII infusion and the risk of viral infections associated with plasma-derived FVIII. Retroviral vector-mediated gene transfer offers the potential for long-term gene expression by virtue of its stable chromosomal integration and lack of viral gene expression. Retroviral vectors for the transfer and expression of a B-domain deleted FVIII gene have been described before.

However, a major problem of FVIII retroviral vectors that has hampered its clinical applications for gene therapy is that both vector titer and FVIII protein production are 100-1000-fold lower in comparison to vectors carrying other cDNA's. Although a 1.2 kb inhibitory region has been identified by deletion analysis of the FVIII cDNA that inhibited FVIII mRNA accumulation by inhibiting transcriptional elongation, conservative mutagenesis of the entire 1.2 kb inhibitory region did not restore viral titer and FVIII expression.

Another problem encountered in attempts at achieving long-term human FVIII expression by ex vivo gene therapy approaches using a variety of primary cells is that access of the engineered cells to the bloodstream (such as by intrasplenic or intravenous injection) is a prerequisite to obtain detectable FVIII levels in the circulation. Cells belonging to the lympho-hematopoietic lineage which can be stably transduced with retroviral vectors ex vivo, do, however, not secrete FVIII protein.

Furthermore, for gene therapy to be successful, high retroviral transduction efficiency and FVIII expression are needed.

The research that led to the present invention showed that human BM stromal cells can be transduced with an intron-based Moloney murine leukemiavirus (MoMLV) retroviral vector expressing a B-domain deleted human factor VIII cDNA (designated as MFG-FVIIIΔB). Transduction efficiencies were increased 10 to 15-fold by phosphate depletion and centrifugation which obviated the need for selective enrichment of the transduced BM stromal cells. This resulted in high FVIII expression levels in transduced human (180 ± 4 ng FVIII/10⁶ cells per 24 hr) and mouse (900 ± 130 ng FVIII/10⁶ cells per 24 hr) BM stromal cells. Pseudotyping of the MFG-FVIIIΔB retroviral vectors with the gibbon ape leukemia virus (GALV) envelope resulted in significantly higher transduction efficiencies (100 ± 20%) and FVIII expression levels (390 ± 10 ng FVIII/10⁶ cells per 24 hr) in transduced human BM stromal cells than with standard amphotropic vectors. This difference in transduction efficiency correlated with the higher titer of the GALV-pseudotyped viral vectors and with the higher GALV receptor (GLVR-1) versus amphotropic receptor (GLVR-2) mRNA expression levels in human BM stromal cells. Human BM stromal cells transduced with GALV-env pseudotyped PG13-F8 vectors using the optimized transduction method as presented in this invention were subsequently infused into immunodeficient SCID NOD mice. Therapeutic human FVIII expression levels were detected in the plasma of these mice well above the levels needed to convert a severe to a moderate hemophilia A patient and persisted for at least more than a week in vivo. This represents the first protocol showing therapeutic levels of FVIII in vivo using BM stromal cells. Most importantly, engraftment of the FVIII-engineered cells was relatively efficient by intrasplenic inoculation and could be achieved even in the absence of myelo-ablation and without having to seed the cells onto artificial fibers or neo-organs. These findings demonstrate the potential of BM stromal cells for gene therapy in general and hemophilia A in particular.

The invention in a specific embodiment thus relates to a method for the ex vivo transduction of mammalian cells, in particular bone marrow cells, more in particular bone marrow stromal cells, which method comprises the steps of:
a) providing an intron-based retroviral vector comprising a B-domain deleted human factor VIII cDNA;
b) pseudotyping the said vector with the Gibbon ape leukemia virus (GALV) envelope;
c) transducing bone marrow stromal cells with the said pseudotyped vector by optionally pre-incubating the cells for a suitable period of time in cell culture medium without phosphate and subsequently adding a vector solution, optionally supplemented with transduction additives, to the cells, followed by centrifugation of the mixture thus obtained; and
d) optionally repeating step c).

In a preferred embodiment of the invention the vector is the MFG-FVIIIΔB vector as described in Dwarki et al. (1995). Information on the MFG-vector is attainable from Riviere et al. (Proc. Natl. Acad. Sci. USA 92:6733-6737, 1995). Details on the nucleotide sequence of the full-length FVIII gene are described in Truett et al., DNA 4:333-349, 1985).

The MFG-FVIIIΔB vector can be pseudotyped with GALV-env by successively transducing PG13 cells (ATCC CRL 10686) therewith. In principle, pseudotyping with GALV-env has been described before for primary human T-cells (Bunnell et al., Proc. Natl. Acad. Sci. USA **92**:7739-7743 (1995)). However, is cannot be derived from his disclosure that pseudotyping would also enhance the efficiency of transduction for stromal cells. Furthermore, Bunnell et al. did not use the FVIII-containing vector of the invention. Details of the pseudotyping according to the invention are given in example 1.

It has been found that in an optimal embodiment of the invention, the transduction protocol comprises both a phosphate starvation and a centrifugation step.

In this application the invention is described in detail referring to the transduction of bone marrow stromal cells for expression of factor VIII. However, the specific steps of ex vivo transduction method can also be used in any combination for the transduction of the same or other cells with the same or other genes.

It is for example possible to use other genes of interest, for example other genes encoding factors secreted into the circulation, in particular factor IX, erythropoietin (EPO), etc.. For the transduction of stromal cells genes may be used that restore stromal function, e.g. for repair of irradiated bone marrow stromal cells after cancer therapy.

Alternative cells to be transduced by the method of the invention are for example BM stromal precursor or BM stromal stem cells which are an integral part of the BM stroma. The advantage of using precursor cells is that the progeny of each transduced stromal precursor cell would also contain the transgene in analogy with transduction of BM hematopoietic stem/progenitor cells. This increases the total number of transduced cells in comparison to the number of transduced cells that arises when terminally differentiated cells are transduced. In addition, transduction of self-renewing precursor stromal cells may lead to prolonged persistence of engineered cells in vivo as compared to transduction of terminally differentiated cells. In principle any cell type can be used, whereas primary cells are preferred.

A vector should contain an expression cassette consisting of either a promoter and an intron or a strong promoter, either one together with the gene of interest. The intron may be located upstream or downstream from the gene. Suitable promoters are viral promoters, e.g. retroviral long terminal repeat (LTR), cytomegalovirus promoter (CMV), simian virus 40 promoter (SV40), adenovirus Major Late Promoter (MLP), etc. or cellular promoters of either housekeeping genes, e.g. mouse or human small nuclear PNA promoter, elongation factor 1α promoter, etc. or tissue-specific promoters that are highly expressed in target tissue.

Suitable introns for use with these promoters are introns from viral genes, e.g. Moloney murine leukemia virus intron etc., from cellular genes, e.g. β-globin intron, Factor VIII or Factor IX intron, ApoA1 intron, α1-antitrypsin intron, etc..

The transduction protocol of the invention is in particular useful for transduction with retroviral vectors. Each step in the transduction protocol provides for an additional increase in the transduction efficiency. Incorporation of all steps in the method is thus preferred.

The general object of the invention of providing an efficient method for ex vivo transduction of mammalian cells is thus achieved according to the invention by the ex vivo transduction by means of a transduction protocol, which method comprises the following elements:
a) the mammalian cell is transduced with a gene of interest;
b) the transduction is effected by means of a retroviral vector;
c) the vector comprises an intron;
d) the transduction protocol comprises pseudotyping of the vector;
e) the transduction protocol comprises a centrifugation step;
f) the transduction protocol comprises phosphate starvation of the cells to be transduced, wherein steps c) to f) are optional and can occur in any possible combination

The above method is suitable for transducing genes encoding proteins which can be of importance for gene therapy, i.e. all genes associated with hereditary disorders or acquired and complex disorders (such as cancer, cardiovascular disease, diabetes etc.) wherein therapeutic effects can be achieved by introducing an intact version of the gene into somatic cells, in particular genes encoding proteins that can be secreted into the circulation (hormones, growth factors, lymphokines and cytokines, interferons, antibodies, complement factors, coagulation factors, enzymes etc.) or genes whose products are not secreted and which may have a direct therapeutic effect on the cell in which they are expressed (enzymes, growth factors or growth factor receptors, signal transducing proteins, cytoskeletal components and other structural proteins, etc.). Particularly in the case of BM stroma, these genes also include genes encoding proteins that are involved in wound healing, bone formation and repair (such as collagen type I which is defective in osteogenesis imperfecta patients) or proteins that influence osteoporosis, arthritis, osteogenesis imperfecta, chondrodysplasia, and hematopoiesis. Other genes also include genes involved in the protection of the BM stroma form the detrimental side-effects on BM stromal function of (i) cancer therapy (such as by chemo- or radiotherapy) or (ii) viral infections (e.g. HIV).

The invention relates in particular to a method in which the gene of interest encodes a gene encoding a factor secreted into the circulation, more in particular a gene encoding a factor involved in blood coagulation. In the specific embodiment described above the mammalian cell is transduced with a gene encoding factor VIII, factor IX, factor X, factor V, factor VII, factor XII, factor XIII, Von Willebrand factor (vWF), tissue factor (TF) and all other proteins directly or indirectly influencing blood coagulation.

The mammalian cell is preferably a primary cell, in particular a primary bone marrow cell. The mammalian cell can also be a bone marrow cell selected from the group consisting of bone marrow stromal stem cells, bone marrow stromal stem cells, bone marrow stromal precursor cells, bone marrow mesenchymal cells, bone marrow hematopoietic stem cells, bone marrow hematopoietic progenitor cells, cells belonging to the lymphohematopoietic lineage, fibroblasts, endothelial cells, chondroblasts, chondrocytes, myoblasts, myocytes, osteoblasts, epithelial cells.

Other cells that can be transduced are mesenthelial cells, keratinocytes, hepatocytes.

Suitable retroviral vectors are Moloney murine leukemia virus, Gibbon ape leukemia virus, Rous sarcoma virus, myeloproliferative sarcoma virus, lentivirus, human foamy virus, human immunodeficiency virus (HIV), simian immunodeficiency virus (SIV), bovine leukemia virus (BLV).

Instead the GALV pseudotyping, the vector can be pseudotyped with other envelopes that utilize the GLVR-1 receptor for viral entry, in particular the 10A1 envelope.

In a specific embodiment, the method comprises:
a) providing an intron-based retroviral vector comprising any gene, in particular a gene that is secretable in the blood stream, more in particular a blood coagulation gene;
b) pseudotyping the said vector with the Gibbon ape leukemia virus (GALV) envelope;
c) transducing bone marrow stromal cells with the said pseudotyped vector by optionally pre-incubating the cells for a suitable period of time in cell culture medium without phosphate and subsequently adding a vector solution, optionally supplemented with transduction additives to the cells, followed by centrifugation of the mixture thus obtained; and
d) optionally repeating step c).

In another embodiment the invention relates to a method for the ex vivo transduction of cells, comprising:
a) providing an intron-based retroviral vector comprising any gene, in particular a gene that is secretable in the blood stream, more in particular a blood coagulation gene;
b) pseudotyping the said vector with the Gibbon ape leukemia virus (GALV) envelope;
c) transducing cells to be transduced, in particular bone marrow stromal precursor cells, with the said pseudotyped vector by optionally pre-incubating the cells for a suitable period of time in cell culture medium without phosphate and subsequently adding a vector solution, optionally supplemented with transduction additives to the cells, followed by centrifugation of the mixture thus obtained; and
d) optionally repeating step c).

A further embodiment of the invention relates to a method for the ex vivo transduction of bone marrow stromal cells, comprising the steps of:
a) providing an intron-based retroviral vector comprising a B-domain deleted human factor VIII cDNA (designated as MFG-FVIIIΔB);
b) optionally pseudotyping the said vector with the Gibbon ape leukemia virus (GALV) envelope; and/or
c) optionally transducing bone marrow stromal cells with the said pseudotyped vector by pre-incubating the cells for a suitable period of time in cell culture medium without phosphate and subsequently adding a vector-containing solution, optionally supplemented with transduction additives to the cell; and/or
d) optionally centrifuging the mixture thus obtained; and/or
e) optionally repeating step c) and d).

Still a further embodiment of the invention relates to a method for the ex vivo transduction of bone marrow stromal cells, comprising the steps of:
a) providing a retroviral vector comprising a B-domain deleted human factor VIII cDNA;
b) optionally pseudotyping the said vector with the Gibbon ape leukemia virus (GALV) envelope; and/or
c) optionally transducing bone marrow stromal cells with the said pseudotyped vector by pre-incubating the cells for a suitable period of time in cell culture medium without phosphate and subsequently adding a vector-containing solution, optionally supplemented with transduction additives to the cell; and/or
d) optionally centrifuging the mixture thus obtained; and/or
e) optionally repeating step c) and d).

An alternative embodiment of the invention relates to a method for the ex vivo transduction of bone marrow stromal cells, comprising the steps of:
a) providing an intron-based retroviral vector comprising a gene of interest;
b) optionally pseudotyping the said vector with the Gibbon ape leukemia virus (GALV) envelope; and/or
c) optionally transducing bone marrow stromal cells with the said pseudotyped vector by pre-incubating the cells for a suitable period of time in cell culture medium without phosphate and subsequently adding a vector-containing solution, optionally supplemented with transduction additives to the cell; and/or
d) optionally centrifuging the mixture thus obtained; and/or
e) optionally repeating step c) and d).

The invention also provides a method for the ex vivo transduction of bone marrow stromal cells, comprising the steps of:
a) providing a retroviral vector comprising a gene of interest;
b) optionally pseudotyping the said vector with the Gibbon ape leukemia virus (GALV) envelope; and/or
c) optionally transducing bone marrow stromal cells with the said pseudotyped vector by pre-incubating the cells for a suitable period of time in cell culture medium without phosphate and subsequently adding a vector-containing solution, optionally supplemented with transduction additives to the cell; and/or
d) optionally centrifuging the mixture thus obtained; and/or
e) optionally repeating step c) and d).

The present invention further relates to the transduced cells thus obtained. The invention in particular relates to bone marrow stromal cells being transduced with an intron-based retroviral vector comprising a B-domain deleted human factor VIII cDNA, which vector has been pseudotyped with the Gibbon ape leukemia virus (GALV) envelope.

The invention according to a further aspect thereof relates to these cells for use in the therapeutical treatment of coagulation disorders, in particular hemophilia A. The therapy of hemophilia A consists for example of returning bone marrow stromal cells, that have been genetically engineered with the gene for factor VIII by the method of the invention to a subject in need of therapy. This type of therapy is called gene therapy. For other types of treatment other types of genes can be transduced to the genetically engineered cells.

The invention also relates to the use of the cells for the preparation of a therapeutic composition for the treatment by means of gene therapy of disorders of the blood, in particular coagulation disorders, more in particular hemophilia A.

Clinical application for hemophilia A using BM stroma requires injection or infusion of about 10⁸ autologous BM stromal cells prepared with the method of the invention to convert a severe to a mild hemophiliac based on an in vitro production of 400 ng/10⁶ cells per 24 hr, assuming that all the engineered cells engraft in vivo and continue to produce these high levels of FVIII. To completely correct the bleeding phenotype, an infusion of at least 7x10⁸ BM stromal cells will be required. At least 10¹⁰ BM stromal cells can be enriched from 1 liter of total BM, which can routinely be obtained from a single BM isolation without any adverse side effects to the donor.

In the literature there are several examples that clinical application of genetically engineered BM stromal cells has a reasonable expectation of success. Ex vivo expansion and subsequent infusion of human BM-derived stromal cells (Lazarus et al., 1995) in phase I clinical trials was not associated with any adverse effects. Retrovirally-marked BM stromal cells have been transplanted efficiently in mice and persisted long-term in vivo even after transplantation in secondary recipients suggesting that stromal precursor cells may have been transduced (Drize et al., Leukemia **6**(3):174S-175S (1992)). Ectopic transplantation of donor-derived stromal cells can replace the host stromal cells in host femurs, suggesting that BM stromal cells mobilized and migrated from the engrafted marrow to the host BM (Ishida et al., J. Immunol. **152**:3119-3127 (1994)). These data were consistent with recent observations showing that human BM stromal cells transduced with a retroviral vector encoding human IL-3 gene, could express physiological levels of IL-3 for several months following transplantation in immunodeficient mice (Nolta et al., Blood **82**:3041-3051 (1994)). Most recently, it has been shown that transiently transfected canine BM stromal cells transiently expressed clotting factor IX or human growth hormone in vitro and after autologous reinfusion in recipient dogs (Hurwitz et al., Hum. Gene Ther. **8**:137-156 (1997)). However, in some studies transplanted stromal cells did not engraft nor participate in the reestablishment of the BM microenvironment. The exact reasons for these differences are not fully understood but may in some cases reflect differences in cell culture methods (Keating et al., Nature **298**:280-283 (1982)).

The persistence and FVIII expression of engineered BM stromal cells have been evaluated in immunodeficient mice. This represents the first protocol showing therapeutic levels of FVIII in vivo using BM stromal cells. The example below gives more details on these studies.

The present invention will be further elucidated in the following example under reference to the figures:

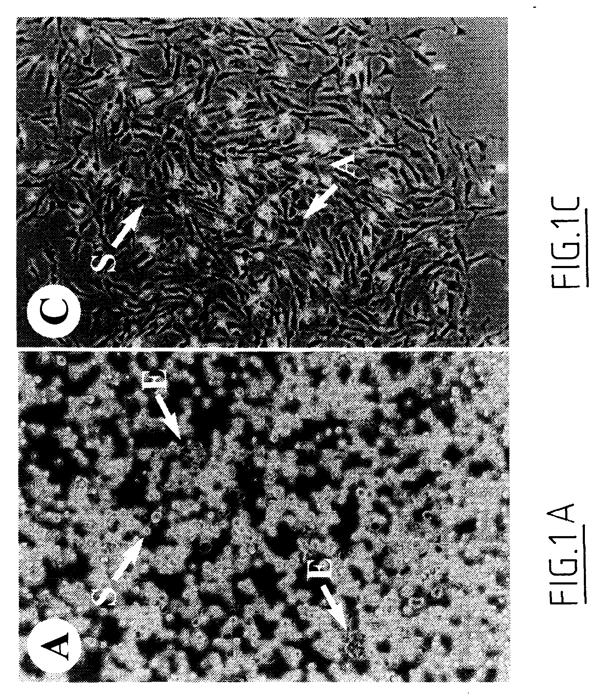

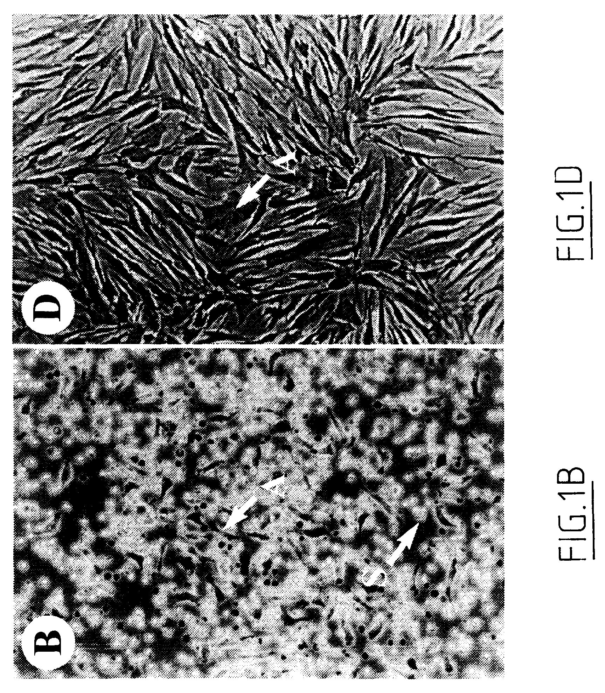

**FIG. 1.** Development of an adherent stromal layer after long-term BM culture. Photomicrographs were taken after 5 days (A), 10 days (B) and 14 days (C) under phase-contrast. A confluent monolayer of subcultured cells at the time of transduction is also shown (D). Adherent stromal cells, suspension cells and erythrocytes are indicated as A, S and E, respectively.

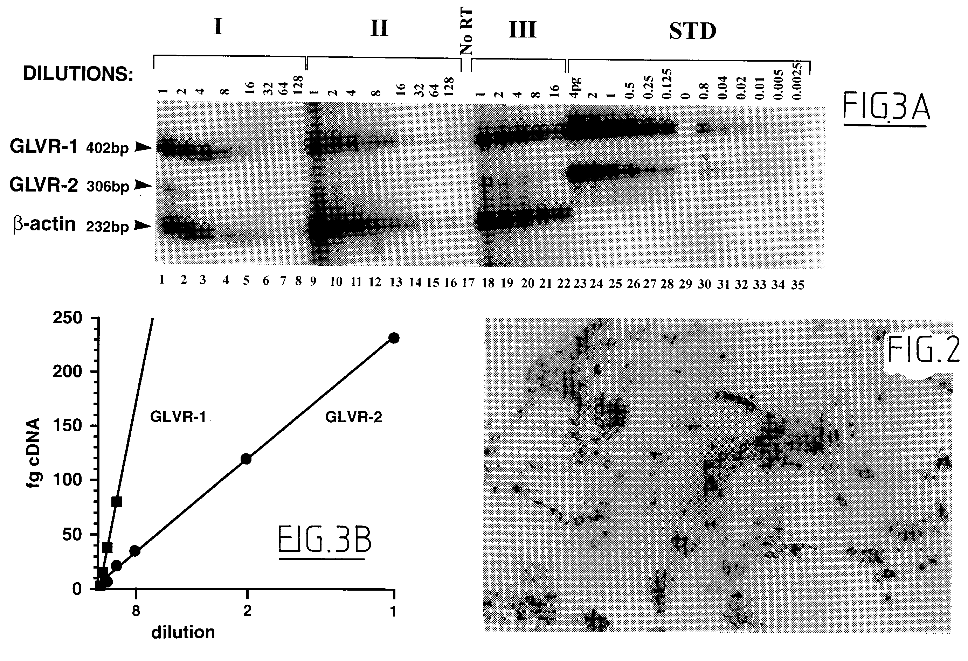

**FIG. 2.** Immunohistochemical staining of human BM stromal cells. Human BM stromal cells were stained for human prolyl 4-hydroxylase, as a fibroblast-specific marker.
**FIG. 3.** Determination of GLVR-1 versus GLVR-2 mRNA expression by quantitative RT-PCR. Purified total RNA (1, 2.5 and 5 µg) from adherent human BM stromal cells was reverse transcribed. Two µl of the reaction mixture containing the cDNA obtained form either 1 µg RNA (1) (lanes 1-8) , 2.5 µg RNA (11) (lanes 9-16) or 5 µg RNA (111) (lanes 18-22) was serially diluted and subjected to PCR with GLVR-1 and GLVR-2 specific primers as described in Materials and Methods. Standards (STD) corresponding to known concentrations of GLVR-1 and GLVR-2 specific amplified fragments subjected to 2-fold serial dilution (ranging from 0-4 pg, lanes 23-35) were used as controls for quantitative comparisons. Negative controls included samples without RT (lane 17) or without template (lane 29). Amplified samples were separated by gel electrophoresis on 1.5 % agarose gels and subjected to Southern analysis with GLVR-1 and GLVR-2-specific oligonucleotides. Quantification was performed using a Phosphorimager after background subtraction (B) using experimental samples that fell within the linear range of the assay corresponding to (lanes 13-16) (A) for GLVR-1 (cDNA obtained from 2.5 µg RNA was diluted 16-128-fold) and (lanes 9-14) (A) for GLVR-2 (cDNA obtained from 2.5 µg RNA was diluted 132-fold).

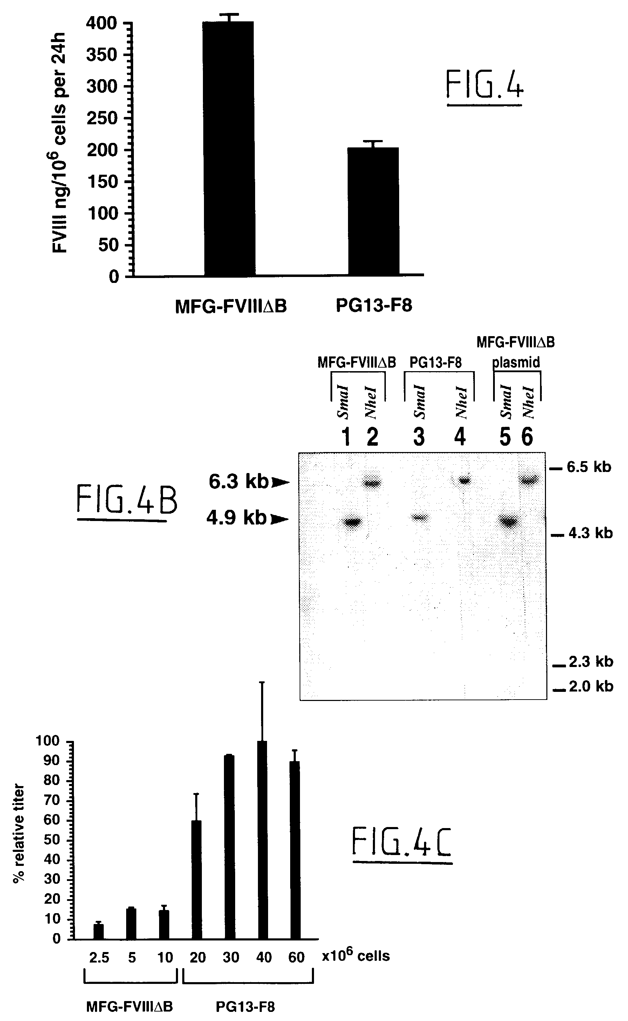

**FIG. 4.** FVIII production (A), Southern blot analysis (B) and titration (C) of FVIII-retroviral vectors pseudotyped with GALV-env (PG13-F8). FVIII production in the PG13-F8 clone #5 compared to the MFG-FVIIIΔB clone #XF2 was quantified with a functional chromogenic FVIII assay (A). Genomic DNA of the MFG-FVIIIΔB producer clone #XF2 (lanes 1,2), the PG 13-F8 producer clone #5 (lanes 3,4) and the control plasmid pMFG-FVIIIΔB (lanes 5,6) were digested with Sma I (lanes 1, 3, 5) or Nhe I (lanes 2, 4, 6) and subjected to Southern blot analysis with a FVIII-specific probe (B). Bands corresponding to proviruses containing only non-deleted FVIII sequences were indicated by arrows; molecular weight markers (in kb) were indicated on the right. Relative viral titer of the MFG-FVIIIΔB and PG13-F8 producer cell clones was determined in function of cell number by RNA dot blot analysis of PEG-precipitated viral vector particles using a FVIII-specific probe as described in the Materials and Methods (C). Quantification was performed using a Phosphorimager after background subtraction.

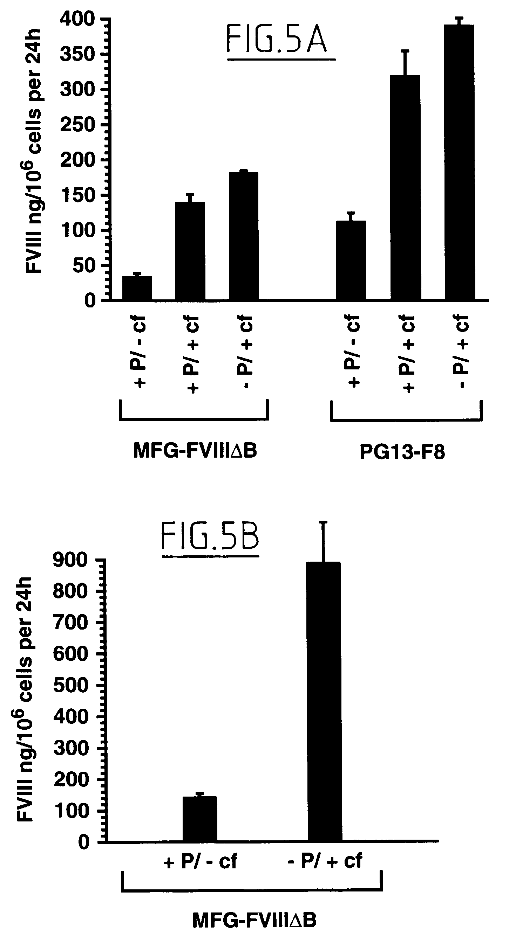

**FIG. 5.** FVIII production in transduced BM stromal cells. Human (A) or mouse (B) BM stromal cells were subjected to 4 successive rounds of transductions with either MFG-FVIIIΔB (A, B) or PG13-F8 (A). The titer and the MOI of PG13-F8 in these transductions (A) was 7-fold higher than MFG-FVIIIΔB. Transductions were performed at 32°C with (+) or without (-) centrifugation (cf) or phosphate (P). FVIII production was determined with a functional chromogenic assay.

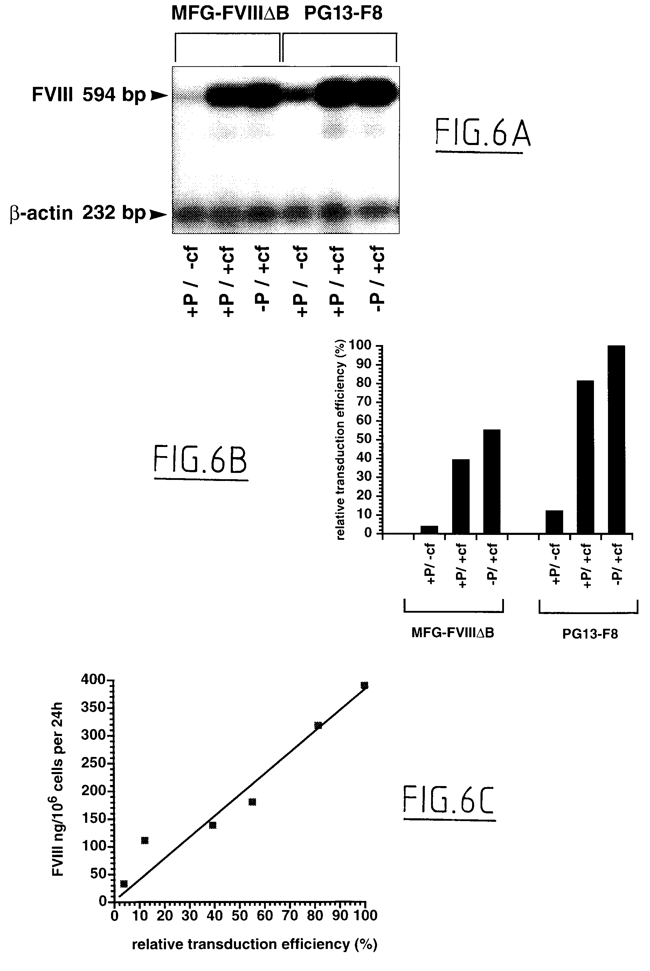

**FIG. 6.** Analysis of transduction efficiency by quantitative PCR/Southern blot analysis. Genomic DNA of the transduced human BM stromal cells was subjected to PCR/Southern blot analysis using primers specific for the FVIII-retroviral vector and β-actin specific primers for normalization. Bands corresponding to the amplified FVIII or β-actin-specific fragments were indicated by arrows (A). The intensities of the PCR-amplified fragments relative to the maximum transduction efficiency (i.e. PG13-F8-transduced after centrifugation and phosphate starvation) were quantified with a Phosphorimager after background subtraction and β-actin normalization yielding the relative transduction efficiencies (%) (B). Transductions were performed with either MFG-FVIIIΔB or PG13-F8 at 32°C with (+) or without (-) centrifugation (cf) or phosphate (P) as indicated (A, B). The FVIII expression was expressed in function of the relative transduction efficiencies of the human BM stromal cells transduced under all conditions tested (C).

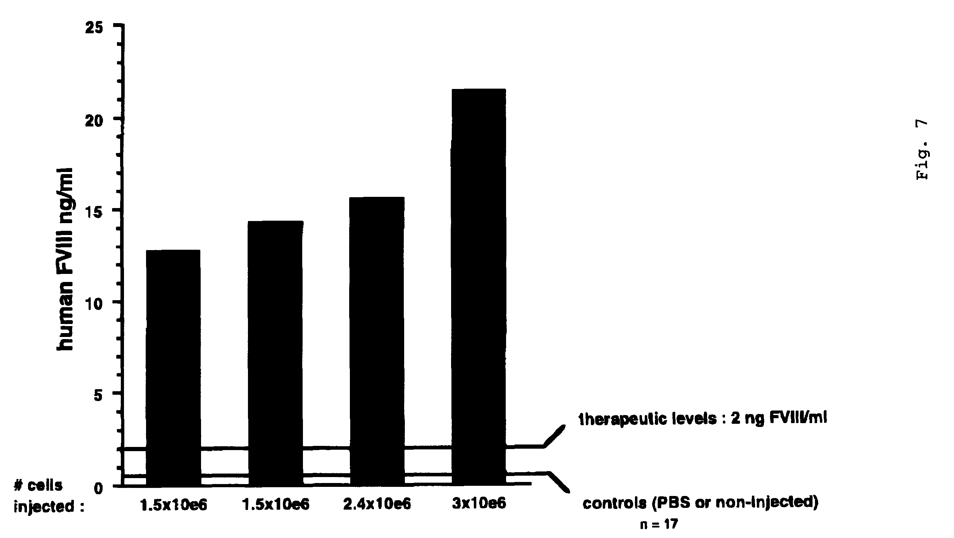

**FIG. 7.** In vivo FVIII expression in NOD-SCID mice. Mice were injected as indicated intrasplenically (i.s.) with 1-3×10⁶ human BM stromal cells transduced with PG13/F8 under optimized conditions. FVIII expression was measured over time with a human FVIII-specific ELISA. The experiment was repeated with BM stroma from unrelated donors and the results of one representative experiment were given.

### EXAMPLES

### EXAMPLE 1

### Transduction of human bone marrow stromal cells with factor VIII

### 1. Introduction

The present example demonstrates the expression of factor VIII in human BM stromal cells and compares the transduction efficiency and expression levels when using various vectors and different transduction protocols.

### 2. Materials and methods

### 2.1 Cell lines and culture conditions

The murine fibroblast-like cell line NIH-3T3, the PG 13 cell line (Miller et al., J. Virol. **65**:2220-2224 (1991), provided by Dr. A.D. Miller - Fred Hutchinson Cancer Research Center, Seattle, WA) and its derivative PG13-F8 (clone #5), the Ψ-CRIP-derived amphotropic producer cell lines MFG-FVIIIΔB (clone # XF2) (Dwarki et al., Proc. Natl. Acad. Sci. USA **92**:1023-1027 (1995), provided by Somatix, Inc., Alameda, CA), the GCsamF8EN producer clones (Chuah et al., Hum. Gene Ther. **6**:1363-1377 (1995)) were cultured in Dulbecco's modified Eagle's medium (DMEM) with 2 mM L-Gln, 100 IU/ml penicillin, 100 µg/ml streptomycin and 10% heat-inactivated fetal bovine serum (designated as D10 medium) (Life Technologies, Merelbeke, Belgium). All cells, including the human and murine BM stromal cells were grown at 37°C in an incubator with 95% humidity and 5% CO₂.

### 2.2 Isolation and in vitro culture of primary human and murine BM stromal cells

Primary human BM stromal cells were obtained from the iliac crest and/or sternum of healthy BM donors since large amounts of enriched bone marrow could be isolated relatively easily by needle aspiration from these sites. BM donors provided their informed consent to participate in the procedure. BM was collected in an equal volume of MyeloCult H5100 medium (StemCell Technologies, Vancouver, Canada) supplemented with 250 U/ml heparin. Erythrocytes were removed by sedimentation for 30 min at room temperature using Plasmasteril (Fresenius, Wilrijk, Belgium). The cells remaining in suspension were washed 3 times in PBS and seeded in 10 cm dishes at densities corresponding to 30x10⁶ cells/15 ml MyeloCult H5100 long term culture medium supplemented with freshly prepared hydrocortisone (10⁻⁶M, Sigma, Bornem, Belgium), 100 IU/ml penicillin, 100 µg/ml streptomycin and 250 ng/ml amphotericin B (Life Technologies) (designated as HBM medium). BM stromal cells started to adhere 5-7 days after BM isolation, at which point 10 ml freshly prepared HBM medium was added. After expanding the BM stromal cells for an additional 7 days, the non-adherent suspension cells were decanted while the adherent stromal layer was trypsinized for transduction or further characterization.

The mouse BM stroma was isolated by sacrificing bnx mice (Harlan, Zeist, the Netherlands) and flushing the femur and tibia with MyeloCult M5300 medium (StemCell Technologies). BM cells harvested either from one femur or two tibiae were cultured in a 10 cm petri dish containing 10 ml of MyeloCult M5300 long-term culture medium supplemented with freshly prepared hydrocortisone hemisuccinate (10⁻⁶M, Sigma), 100 IU/ml penicillin, 100 µg/ml streptomycin and 250 ng/ml amphotericin B (Life Technologies) (designated as MBM medium). Adherent mouse BM stromal cells were obtained by growing the cells for 3-7 days. After expanding the BM stromal cells for 7 days, the non-adherent suspension cells were decanted while the adherent stromal layer was trypsinized for transduction.

### 2.3 Vectors

The MFG-FVIIIΔB and the GCsamENF8 splicing vectors were described previously (Dwarki et al., 1995, supra; Chuah et al., 1995, supra) and can be reconstructed quite easily from these descriptions. In these vectors, the B-domain deleted FVIII gene was driven from the 5' MoMLV LTR and was cloned downstream of the MoMLV intron used to generate subgenomic env mRNA. In contrast to the GCsamF8EN vector, a Kozak consensus sequence for translational initiation was introduced in the MFG-FVIIIΔB vector and the 3' untranslated region (UTR) of the FVIII gene was deleted. The MFG-FVIIIΔB vector lacked a neo^{R} selectable marker, whereas the GCsamF8EN vector expressed FVIII and the neomycin phosphotransferase II (NPTII) proteins from a single polycistronic transcript that was driven from the 5' MoMLV LTR by virtue of the internal ribosome entry site (IRES).

### 2.4 Generation and characterization of FVIII retroviral vectors pseudotyped with GALV-env

Viral supernatant was first collected over 24 hr from a confluent plate of MFG-FVIIIΔB producer cells (clone #XF2) and filtered through a 0.45 µm filter to remove residual producer cells. To generate the MoMLV/GALV-env pseudotyped retroviral vectors, 8x10⁶ PG13 cells were subjected to successive daily transductions with 5 ml of MFG-FVIIIΔB viral vector-containing conditioned medium in the presence of polybrene (8 µg/ml, Sigma). The resulting producer cells were designated as PG13-F8 and individual clones were obtained by limiting dilution. FVIII production by each of the individual PG13-F8 clones was quantified using a functional chromogenic assay as described below. Clones that expressed the highest levels of FVIII were further screened for viral production by RNA dot blot analysis (see below) and subsequently subjected to Southern blot analysis as described previously (Sambrook et al., Molecular Cloning 16-32 (1989), Chuah et al., 1995, supra) to exclude the presence of rearranged proviral sequences. Briefly, genomic DNA was extracted with the high pure PCR template preparation kit (Boehringer, Mannheim, Germany) and 23 µg of DNA was restricted with Sma I or Nhe I. Hybridizations were performed by probing the Southern blot membrane with a FVIII-specific probe corresponding to a random primed 1095 bp Bgl II - Spe I restriction fragment of plasmid pMT2LA. The membranes were washed stringently at 65°C in 2xSSC and 0.1% SDS for 30 min, followed by 0.5xSSC and 0.2% SDS for an additional 30 min.

### 2.5 FVIII quantification

FVIII activity in the transduced stromal cells and the viral producer cell clones was quantified by measuring the FVIII-dependent generation of factor Xa from factor X using a chromogenic assay (Coatest FVIII, Chromogenix, Molndal, Sweden) as described previously (Chuah et al., 1995, supra). Briefly, 24 hr-conditioned culture medium was harvested in phenol-red free media to avoid calorimetric interference in the FVIII chromogenic assay. Human plasma purified FVIII (Octapharma, Langenfeld, Germany) of known activity was used as a FVIII standard and 1 U was defined as 200 ng FVIII/ml. The lowest level of detectable FVIII was 0.01-0.03 ng/ml and media containing heat-treated FBS did not yield any detectable FVIII activity.

### 2.6 Viral production and titration

Supernatant containing retroviral vector particles was obtained by seeding 4x1O⁶ producer MFG-FVIIIΔB and 30x10⁶ producer PG13-F8 in 10 ml of D10 per 75 cm² flask, unless indicated otherwise. These cells were grown at 32°C and the supernatant was harvested after 24 hr. Supernatants were aliquoted and immediately frozen on dry ice prior to storing at -80°C until use. Vector titer in the culture medium was determined by RNA dot blot analysis as described previously (Yang et al, Hum. Gene Ther. **6**:1203-1213 (1995)). Hybridizations were performed by probing the membrane with a FVIII-specific probe corresponding to a random primed 1095 bp Bgl II - Spe I restriction fragment of pMT2LA. The membranes were washed stringently at 65°C in 2xSSC for 30 min followed by 0.5xSSC for an additional 30 min. After background subtraction, signal intensities were quantified using a Phosphorimager (Molecular Dynamics, Sunnyvale, CA). Additional controls consisted of serially diluted viral vector supernatants with known functional titer based on vectors containing a neo^{R} gene (GCsamF8EN). Functional titers expressed as G418^{R} cfu/ml were determined by transduction of NIH-3T3 cells as described previously (Chuah et al., 1994 and Chuah et al., 1995, supra).

### 2.7 Immunohistochemical analysis of human BM stroma

Human BM stromal cells were seeded at a concentration of 10⁴ cells per well in 0.4 ml Iscove's modified Dulbecco's medium (IMDM) supplemented with freshly prepared hydrocortisone (10⁻⁶M, Sigma), 10% heat-inactivated FBS, 2 mM L-glutamine, 100 IU/ml penicillin, 100 µg/ml streptomycin and 250 ng/ml amphotericin B (designated as I10 medium) in 8-chamber slides (Life Technologies) and incubated at 37°C. Medium was removed from chamber slides containing confluent cells followed by fixation with acetone at -20°C for 5 min. Fixed cells were washed twice in Tris saline buffer (TSB) pH 7.6 (100 mM Tris, 150 mM NaCl, 2 g/1 Merthiolate, 10 ml/1 triton X-100 ). After removing excess TSB, the cells were incubated for 30 min in TSB containing 0.3% H₂O₂ to consume potential endogenous peroxidase activity, and subsequently washed twice with TSB.

Immunostaining was performed by first incubating the cells for 30 min in 100 µl 5-fold diluted pre-immune rabbit serum (Dako, Glostrup, Denmark) for the fibroblast-specific immunostaining or pre-immune swine serum for the endothelial cell-specific immunostaining (Dako), after which supernatant was removed. For the fibroblast-specific immunostaining, the cells were incubated overnight with a monoclonal mouse antibody that reacted with human prolyl 4-hydroxylase (clone 5B5, 155 µg/ml Dako) diluted 1/50-1/100 in TSB. The next day, cells were washed twice in TSB and incubated for 1 hr in 100 µl diluted (1/400) biotinylated rabbit anti-mouse immunoglobulins (800 µg/ml, Dako). For the endothelial-cell specific immunostaining, the cells were incubated with a polycolonal rabbit antibody specific for von Willebrand factor (vWF) (Dako) diluted 1/300 in TSB. The next day, cells were washed twice in TSB and incubated for 1 hr in 100 µl diluted (1/500) biotinylated swine anti-rabbit immunoglobulins (Dako). Subsequently, the cells were washed twice for 5 min in Tris pH 7.5 (500 mM) and incubated for 1 hr with avidin-biotin complex peroxidase (ABC-PO, Dako). After washing twice for 5 min in Tris, cells were stained with 240 ml Tris containing 30 mg diaminobenzidine (DAB) and 78 µl H₂0₂ (30%). The reaction was terminated by washing the cells in TSB and counterstaining was performed using Harris' hematoxylin.

After dehydrating the cells in gradually increasing concentrations of ethanol and xylol, slides were embedded in DPX (Prosan, Gent, Belgium). Additional controls without pre-immune serum, primary or secondary antibodies and NIH-3T3 fibroblasts and human umbilical vein endothelial cells (HUVEC) were also included as controls in each experiment.

### 2.8 Analysis of GLVR-1 and GLVR-2 expression

The relative GLVR-1 and GLVR-2 mRNA expression levels in the human BM stromal cells were determined by reverse transcriptase-polymerase chain reaction (RT-PCR). Total RNA was first purified using the chaotropic Trizol method followed by phenol-chloroform extraction and isopropanol precipitation (Chomczynski, P., Biotechniques **15**:532-534, 536-537 (1993)). The precipitated RNA was then redissolved in H₂0 and spectrophotometrically quantified. The first strand cDNA was synthesized starting from 1, 2.5 and 5 µg purified total RNA using a Superscript II reverse transcriptase kit (Life Technologies). The cDNA was subsequently amplified directly by PCR using 2 µl of the reaction mixture, that was subjected to serial 2-fold dilutions. PCR was performed with a Techne/Progene thermocycler using Taq polymerase and oligonucleotide pairs that discriminated specifically between GLVR-1 (5'-GCAGTTTTCTGTGCCCTTATCGTC-3' and 5'-GGAGTTTATTTGGTTGCTGACGG-3') and GLVR-2 (5'-TTCAGGAAGCAGAGTCCCCAGT-3' and 5'-TGTCGATGTGGATTTTGCAG-3'). PCR was performed by denaturation for 5 min at 94°C, followed by 26 cycles of 45 sec at 94°C, 1 min at 60°C, 2 min at 72°C and a final extension for 7 min at 72°C. The RT-PCR reaction products obtained from 1 µg, 2.5 µg and 5 µg RNA were subjected to serial 2-fold dilution (ranging from 2- to 128-fold dilution) to ensure that detection of the PCR amplified fragments fell within the linear range of the quantitative PCR.

Standards corresponding to known concentrations of GLVR-1 and GLVR-2-specific amplified fragments subjected to 2-fold serial dilution (ranging from 0 to 4 pg) were used as controls for quantitative comparisons. These standards were generated by amplifying RT-PCR products from human T-cells using the same PCR conditions as for the experimental samples, except that the cDNA was subjected to 35 rounds of amplification. These amplified fragments were then purified by Geneclean (Westburg, Leusden, The Netherlands) and the amounts were quantified spectrophotometrically. Amplified samples were separated by gel electrophoresis on 1.5 % agarose gels and transferred to Hybond (Amersham, UK) membranes by Southern blotting as described previously (Sambrook et al., 1989, supra, Chuah et al., 1994, supra). Following prehybridization, the membranes were hybridized overnight to ³²P-end labelled GLVR-1 and GLVR-2-specific oligonucleotides that were labelled using polynucleotide kinase as described previously (Sambrook et al., 1989, supra) followed by stringent washings and quantitation using a Phosphorimager apparatus.

### 2.9 Transduction of BM stromal cells

Human and murine BM stromal cells were seeded at a density of 10⁵ cells/ml per well in a 6-well plate containing 1 ml I10 medium supplemented with freshly prepared hydrocortisone (10⁻⁶ M, Sigma) for human stroma or hydrocortisone-hemisuccinate (10⁻⁶ M, Sigma) for mouse stroma. The next day, the I10 medium was aspirated and BM stromal cells were washed once with 5 ml of PBS followed by transduction with the amphotropic MFG-FVIIIΔB vector (for murine and human stroma) or the GALV-env pseudotyped PG13-F8 vector (for human stroma) under standard or optimized conditions. Standard conditions involved overnight incubation of the stromal cells with vector-containing supernatant at 32°C by virtue of the increased stability of retroviral vectors at 32°C versus 37°C. Vector-containing supernatants were supplemented with 4 µg/ml protamine sulphate and hydrocortisone (or hydrocortisone-hemisuccinate). Under optimized conditions, vector-containing supernatant with protamine sulphate and hydrocortisone (or hydrocortisone-hemisuccinate) was added to the cells followed by a centrifugation step at 32°C for 1 hr at 1400 g and an overnight incubation at 32°. Cells were returned to 37°C the next day. For some transductions, a phosphate starvation step was included that involved a 9-10 hr incubation of the stromal cells in I10 medium containing IMEM without phosphate. A total of 4 rounds of transductions were performed successively under the same conditions over the next 4 days followed by two washings with PBS.

### 2.10 Analysis of transduction efficiency by quantitative PCR and Southern blot

High molecular weight genomic DNA was isolated from the transduced BM stromal cells using the high pure PCR template preparation kit (Boehringer, Mannheim, Germany). To determine transduction efficiency, PCR was performed with a Techne/Progene thermocycler using Taq polymerase and oligonucleotide pairs specific for the FVIII cDNA in the MFG-FVIIIΔB or GCsamF8EN retroviral vectors (5'-GAGCTCTCCACCTGCTTCTTTCTG-3' and 5'CCCTTCTCTACATACTAGTAGGGC-3') yielding a specific 594 bp PCR product. For normalization, β-actin-specific primers (5'-CATTGTGATGGACTCCGGAGACGG-3' and 5'-CATCTCCTGCTCGAAGTCTAGAGC-3') were added to the PCR reaction mixture yielding a 232 bp β-actin specific PCR product. A producer clone transduced with GCsamF8EN and containing 6 integrated proviral copies was used as a control. PCR was performed by denaturation for 8 min at 95°C, followed by 28 cycles of 1 min at 95°C, 1 min at 59°C, 2 min at 72°C and a final extension for 5 min at 72°C. Amplified samples were separated by gel electrophoresis on 1.5% agarose gels and transferred to Hybond (Amersham) membranes by Southern blotting as described previously (Sambrook et al., 1989, supra, Chuah et al., 1994, supra). Following prehybridization, the membranes were hybridized overnight to ³²P-end labelled FVIII and β-actin-specific oligonucleotides that were labelled using polynucleotide kinase as described previously (Sambrook et al., 1989, supra). Membranes were washed under stringent conditions (65°C in 2 x SSC for 30 min followed by 0.5 x SSC for an additional 30 min) and quantitation was performed using a Phosphorimager.

### 2.11 Statistical analysis

The results are represented as mean ± standard deviation of triplicate experiments. The significance of the difference was determined using Student's t-test for unpaired values.

### 3. Results

### 3.1 Characterization of human BM stromal cells

The kinetics of the development of an adherent BM stromal cell layer of different BM donors did not vary significantly and a representative example is given is Fig. 1. During the first 5 days (Fig. 1A), most BM cells remained in suspension except for the presence of adherent macrophage-like cells. Less than 5% of the suspension cells were erythrocytes. Cells with a spindle-like morphology started to adhere between day 6-10 (Fig. 1B) whereas suspension cells gradually disappeared from the cultures. The adherent cells continued to proliferate and formed cell aggregates that extended to generate patchy areas of an adherent layer (Fig. 1C). The hemopoietic foci and residual suspension cells were removed from the cultures (Figs. 1C, 1D) and the adherent cells were trypsinized and expanded to generate an adherent stromal layer (Fig. 1D). More than 95% of the adherent stromal cells exhibited the distinctive spindle-like morphology that was retained even after long-term culture (at least 4 weeks).

Since BM contains endothelial and fibroblast-like cells, the identity of the spindle-like stromal cell layers was confirmed by immunohistochemical analysis. Immunostaining for fibroblasts was performed using a murine monoclonal antibody against the β-subunit of human prolyl 4-hydroxylase which catalyses the hydroxylation of proline residues in collagens to hydroxyproline. The majority of the stromal cells (84 ± 8.0 %) stained for the prolyl 4-hydroxylase antigen indicating that they are fibroblastic (Fig. 2A) whereas no staining was observed when the primary or secondary antibody was omitted and when using the NIH-3T3 mouse cell fine as a negative control, as expected (data not shown). Immunostaining for endothelial cells with a polyclonal antibody against human vWF only revealed aspecific nuclear staining (data not shown), indistinguishable from negative control in which the primary antibody had been omitted. In contrast, human umbilical vein endothelial cells that were used as positive controls (data not shown) exhibited very strong cytoplasmic staining with the vWF-specific polyclonal antibody (92 ± 3.1%). These data indicate that the BM stromal layer in long-term BM culture conditions consisted mainly of fibroblast-like cells.

### 3.2 Comparison of GLVR-1 and GLVR-2 expression in human BM stromal cells

To correlate the efficiency of transduction of human BM stromal cells by amphotropic MoMLV or GALV-env pseudotyped retroviral vectors with the relative abundance of GLVR-1 (GALV-env ) and GLVR-2 (amphotropic) receptor on the target cells their relative expression levels were determined. Since monoclonal antibodies are currently not available, relative expression levels were determined at the transcriptional level by quantitative RT-PCR/Southern blot analysis, using GLVR-1 and GLVR-2 specific primer-pairs that amplified a non-homologous region of 402 and 306 bp respectively (Fig. 3A). A linear standard curve (correlation coefficient: r² = 0.98) was obtained for known amounts of GLVR-1 and GLVR-2 cDNA fragments ranging between 0 to 125 fg (Fig. 3A, lanes 28-35). Detection of GLVR-1 and GLVR-2 specific PCR-amplified fragments from the BM stromal cells fell within the linear range of the assay for the RT-PCR reaction products obtained from 1 µg RNA (Fig. 3A, lanes 1-8) and 2.5 µg RNA (Fig. 3A, lanes 9-16) (r² = 0.97-0.99). No PCR amplified product could be detected in the negative controls that did not contain RT (Fig. 3A, lanes 17) excluding amplification from potentially contaminating genomic DNA. As expected, no PCR product was observed in the absence of GLVR-1 and GLVR-2 cDNA templates (Fig. 3A, lane 29).

Comparative analysis of GLVR- 1 and GLVR-2 expression in human BM stromal cells determined from the amounts of GLVR-1 and GLVR-2-specific cDNA fragments at different dilutions indicated that GLVR- 1 expression was 6-fold higher than GLVR-2. In control experiments with human CD4⁺ T-lymphocytes, GLVR-1 was expressed at high levels whereas only very low levels of GLVR-2 specific PCR fragments could be detected (data not shown).

### 3.3 Generation of the GALV-env pseudotyped FVIII-retroviral vector and comparison with the amphotropic MoMLV vector

In view of the higher GLVR-1 (GALV-env) versus GLVR-2 (amphotropic) receptor expression levels in human BM stromal cells (Fig. 3), GALV-env pseudotyped FVIII-retroviral vectors were generated (designated as PG13-F8) in an attempt to obtain high transduction efficiencies. Six out of the 23 PG13-F8 clones that were screened, expressed functional FVIII ranging from 5 to 200 ng FVIII/10⁶ cells per 24 hr. PG13-F8 clone #5 expressed 200 ng FVIII/10⁶ cells per 24 hr, which is 2-fold (p < 0.001) lower than the MFG-FVIIIΔB producer clone #XF2 (410 ± 9 ng FVIII/10⁶ cells per 24 hr) (Fig. 4A). Southern blot analysis of high producer PG13-F8 clones using different restriction enzymes confirmed the presence of non-rearranged, intact FVIII-proviral sequences and absence of rearranged proviral DNA as shown for PG13-F8 clone #5 (Fig. 4B). Since the MFG-FVIIIΔB did not contain a selectable marker, viral titer was determined by RNA dot blot analysis. Using GCsamF8EN for calibration with a functional titer of 5x10⁵ G418^{R} cfu/ml, RNA dot blot analysis yielded an equivalent average functional titer for MFG-FVIIIΔB of 6 ± 3x10⁴ G418^{R} cfu/ml based on 23 independent batches of viral supernatant. The titer of the PG 13-F8 clone #5 was equivalent to (1.8 ± 0.6)x10⁵ cfu/ml based on 17 independent batches of viral supernatant. Significantly higher (p < 0.001) viral titers could consistently be achieved with the PG13-F8 producer clone #5 than with the best producer MFG-FVIIIΔB XF2 clone. The main reason for this difference is that the PG13-F8 clone #5 could be grown at higher cell densities as compared to the MFG-FVIIIΔB XF2 clone (Fig. 4C). When cells were seeded at similar densities, both the PG13-F8 pseudotyped producer cell clone #5 and the MFG-FVIIIΔB clone #XF2 exhibited similar titers.

### 3.4 Comparison of FVIII expression in BM stromal cells transduced with GALV-env pseudotyped or the amphotropic MoMLV FVIII retroviral vectors under standard and optimized conditions

Human BM stroma transduced with MFG-FVIIIΔB vector-containing supernatant under standard conditions expressed 33 ± 6 ng FVIII/10⁶ cells per 24 hr (Fig. 5A). BM stromal cells transduced with GALV-env pseudotyped vector-containing supernatant from the PG13-F8 clone #5 expressed 110 ± 13 ng FVIII/10⁶ cells per 24 hr. This represented a significant (p < 0.001) 3 to 4-fold increase in FVIII production than when BM stromal cells were transduced with the amphotropic MFG-FVIIIΔB vector. The higher FVIII expression levels of PG13-F8 versus MFG-FVIIIΔB transduction was also confirmed in 3 independent experiments using BM from different donors, even when the multiplicities of infection (MOI) of MFG-FVIIIΔB and PG13-F8 were normalized (data not shown).

Transduction of human BM stroma with the MFG-FVIIIΔB retroviral vector using an optimized transduction protocol that involved centrifugation in combination with phosphate starvation yielded 180 ± 4 ng FVIII/10⁶ cells per 24 hr (Fig.5A), representing a significant 6-fold increase (p < 0.001) of FVIII expression over the standard method. Similarly, human BM stroma transduced under optimized conditions with the GALV-env pseudotyped (PG13-F8) retroviral vector expressed 390 ± 12 ng FVIII/10⁶ cells per 24 hr (Fig. 5A), a significant 3 to 4-fold increase (p < 0.001) over the standard method. These observations were consistent in 3 independent experiments using BM stroma obtained from different donors and the results of one representative experiment are depicted.

In murine BM stromal cells, the optimized transduction protocol with the MFG-FVIIIΔB vector also yielded significantly higher FVIII expression levels (p < 0.001) (900 ± 130 ng FVIII/10⁶ cells per 24 hr) as compared to the standard protocol (140 ± 14 ng FVIII/10⁶ cells per 24 hr) (Fig. 5B). The FVIII expression levels in mouse BM stromal cells transduced with the MFG-FVIIIΔB vector were consistently higher than in human BM stroma transduced with the same vector possibly due to the higher proliferative capacity of the mouse BM stroma at the inception of transduction, resulting in higher transduction efficiencies and FVIII expression levels.

The individual contributions of either phosphate starvation or centrifugation to the increase in FVIII expression were also evaluated. Human BM stromal cells transduced with the MFG-FVIIIΔB retroviral vector-containing supernatant, yielded FVIII expression levels of only 33 ± 6 ng/10⁶ cells per 24 hr when transductions were performed without centrifugation, but 140 ± 13 ng/10⁶ cells per 24 hr with centrifugation (Fig.5A) (p < 0.001). Similarly, when PG13-F8 retroviral vector-containing supernatant was used, FVIII expression was significantly higher (p < 0.001) with centrifugation (320 ± 37 ng FVIII/10⁶ cells per 24 hr) than without (110 ± 13 ng FVIII/10⁶ cells per 24 hr) (Fig.5A). Hence, the centrifugation step alone (without phosphate starvation) strongly increased FVIII expression levels in transduced human BM stromal cells.

In contrast, a relatively moderate increase in FVIII expression levels was observed after phosphate starvation. Human BM stromal cells transduced with the MFG-FVIIIΔB retroviral vector-containing supernatant yielded FVIII expression levels of 140 ± 13 ng FVIII/10⁶ cells per 24 hr without phosphate starvation whereas FVIII expression levels were significantly higher (p < 0.001) with phosphate starvation (180 ± 4 FVIII ng/10⁶ cells per 24 hr) (Fig.5A). When PG13-F8 retroviral vector-containing supernatant was used, FVIII expression without phosphate starvation corresponded to 320 ± 37 ng FVIII/10⁶ cells per 24 hr whereas significantly higher (p < 0.05) levels were obtained after phosphate starvation (390 ± 12 ng FVIII/10⁶ cells per 24 hr) (Fig. 5A). Hence, phosphate starvation had contributed to a significant but only moderate increase in FVIII expression levels in transduced human BM stromal cells.

In conclusion, the development of the GALV-env pseudotyped MFG-FVIIIΔB retroviral vector in combination with the use of an optimized transduction protocol (comprising centrifugation and phosphate starvation) has led to an overall 12-fold increase in FVIII expression levels in transduced human BM stromal cells as compared to transductions performed under standard conditions with the amphotropic MoMLV vector.

### 3.5 Analysis of transduction efficiency in BM stromal cells transduced with GALV-env pseudotyped or amphotropic FVIII retroviral vectors under standard and optimized conditions

Quantitative PCR/Southern blot analysis was performed to determine whether the differences in FVIII expression in transduced human BM stromal cells reflected actual differences in transduction efficiencies. The PCR was repeated 3 times and a representative experiment was shown in Fig. 6.

Human BM stroma were transduced 2 to 3-fold (p < 0.05) more efficiently with the GALV-env pseudotyped PG13-F8 retroviral vector-containing supernatant than with the amphotropic MFG-FVIIIΔB vector in standard and optimized conditions (Fig. 6A & B). The optimized transduction method led to a significant (p < 0.005) 10 to 15-fold increase in transduction efficiency compared to the standard method when the stroma was transduced with the MFG-FVIIIΔB or PG13-F8 vector (Fig. 6A & B). The differences in transduction efficiency correlated with the differences in FVIII expression levels in the transduced BM stromal cells (Fig. 6C).

The individual contributions of either phosphate starvation or centrifugation to the increase in transduction efficiency was also evaluated. A significant 8 to 10-fold increase (p < 0.05) in transduction efficiency was observed when BM stromal cells were subjected to centrifugation during the transductions with the MFG-FVIIIΔB and PG13-F8 vectors than when the centrifugation step was omitted (Fig. 6A & B). In contrast, a relatively slight increase of 1.4-fold in transduction efficiency was observed after phosphate starvation (Fig. 6A & B). Hence, the contribution of the centrifugation during the transduction was more important to the overall increase in transduction efficiency than the phosphate starvation, which is consistent with the differences in FVIII expression levels.

In conclusion, the development of the GALV-env pseudotyped MFG-FVIIIΔB retroviral vector in combination with the use of an optimized transduction protocol (comprising centrifugation and phosphate starvation) has led to an overall 20 to 30-fold increase in transduction efficiency in transduced human BM stromal cells as compared to transductions performed under standard conditions with amphotropic vectors. Human BM stroma transduced with PG13-F8 under optimized conditions contained an average of 1.0 ± 0.2 integrated FVIII-proviral copies/cell, by calibrating against retroviral producer clone with know FVIII proviral copy number. Assuming that each cell contained only one FVIII-provirus, this would imply that 100 ± 20% of the cells had been transduced.

### 4. Discussion

In the present example it has been demonstrated that human or mouse BM stromal cells can be exploited as an alternative BM-derived target cell for hemophilia A gene therapy since they could express relatively high levels of human FVIII (400-900 ng/10⁶ cells per 24 h) when transduced with FVIII retroviral vectors. The high levels of FVIII could be attributed to the use of an intron-based vector, the development of an optimized transduction protocol and the generation of a GALV-env pseudotyped FVIII retroviral vector. Hence, a large number of FVIII expressing primary BM stromal cells could be obtained while obviating the need to enrich for transduced cells by selection and without inducing stromal cell proliferation by supplementing high doses of exogenous purified growth factors. These improvements shorten the in vitro culture period of the BM stromal cells that are thus more likely to retain their original properties. Furthermore, since selective enrichment of transduced cells was not needed, it was not necessary to include a neo^{R} selectable marker in the vector.

### EXAMPLE 2

### In vivo FVIII expression in mice

### 1. Introduction

Human BM stromal cells were first transduced with FVIII retroviral vectors in vitro using the optimized transduction method with GALV-env pseudotyped MFG-FVIIIΔB vectors as presented in this invention and were subsequently infused into immunodeficient SCID NOD mice. Therapeutic human FVIII expression levels were detected in the plasma of these mice well above the levels needed to convert a severe to a moderate hemophilia A patient and persisted for at least 1 week in vivo. This represents the first in vivo study showing therapeutic levels of FVIII using BM stromal cells. Most importantly, engraftment of the FVIII-engineered cells could be achieved even in the absence of myelo-ablation and without having to seed the cells into artificial fibers or neo-organs.

One advantage of the present method is that no myeloablation is required. Because of this, the gene therapeutic method described herein is clinically acceptable for hemophilia patients. Furthermore, the in vivo expression levels and kinetics are comparable to what has been reported previously using neo-organs (Dwarki et al., 1995, supra).

### 2. Materials and methods

### 2.1 Transduction of BM stromal cells

Human BM stromal cells were seeded at a density of 10⁵ cells/ml per well in a 6-well plate containing 1 ml I10 medium supplemented with freshly prepared hydrocortisone (10⁻⁶ M, Sigma) for human stroma. The next day, the I10 medium was aspirated and BM stromal cells were washed once with 5 ml of PBS followed by transduction with the GALV-env pseudotyped PG13-F8 vector under optimized conditions. Vector-containing supernatant was collected at 32°C. Vector containing supernatants were supplemented with 4 µg/ml protamine sulphate and hydrocortisone and added to the cells followed by a centrifugation step at 32°C for 1 hr at 1400 g and an overnight incubation at 32°C. Cells were returned to 37°C the next day. A phosphate starvation step was included that involved a 9-10 hr incubation of the stromal cells in I10 medium containing IMEM without phosphate. A total of 8 rounds of transductions were performed successively under the same conditions over the next 2 weeks followed by two washings with PBS.

### 2.2 Injection of BM stromal cells

BM stromal cells that were stably transduced with the PG13-F8 retroviral vector were trypsinized, washed with PBS and resuspended at a cell density of 7.5 to 15x10⁶ per ml of PBS. Four to 5 weeks old male NOD-SCID recipient mice were injected intra-splenically through a small incision using a 27G needle with 1.5 to 3x10⁶ BM stromal cells per 200 µl. Control mice received an intrasplenic injection of 200 µl PBS.

### 2.3 Detection of FVIII expression in vitro and in vivo

FVIII in vitro activity in the transduced stromal cells and the viral producer cell clones was quantified by measuring the FVIII-dependent generation of factor Xa from factor X using a chromogenic assay (Coatest FVIII, Chromogenix, Molndal, Sweden) as described previously (Chuah et al., 1995, supra). Briefly, 24 hr-conditioned culture medium was harvested in phenol-red free media to avoid colorimetric interference in the FVIII chromogenic assay. Human plasma purified FVIII (Octopharma, Langenfeld, Germany) of known activity was used as a FVIII standard and 1 U was defined as 200 ng FVIII/ml. The lowest level of detectable FVIII was 0.01-0.03 ng/ml and media containing heat-treated FBS did not yield any detectable FVIII activity.

FVIII in vivo expression was determined by collecting mouse plasma at regular intervals and performing a human FVIII-specific ELISA. A 96-well microtite plate was coated with 2 monoclonal antibodies to human FVIII which do not cross-react with mouse FVIII: N77110M (Biodesign, Kennebunkport, ME) and ESH2 (American Diagnostica, Greenwich, CT) at a concentration of 25 µg/ml. After incubation overnight at 4°C, the plate was washed three times with PBS. Blocking agent (PBS, 10% horse serum, 1 mM CaCl₂) was added to each well, the plate was incubated for 2 hr at room temperature, and the wells were washed with 200 µl PBS, 0.05% Tween 20 three times. Plasma samples were diluted 1:5 in TNTC buffer (50 mM Tris pH 7.2, 5 mM CaCl₂, 0.1% Tween 20, 0.5 M NaCl) and 100 µl was added into each well. Mouse plasma spiked with known concentrations of serially diluted purified human FVIII was used to generate a standard curve.

The plate was incubated for 1 hr at 37°C, the wells were washed 3 times with PBS, 0.05% Tween 20 and 100 µl of the second antibody was added. The second antibody was serum from a hemophiliac with a high inhibitor titer, diluted 1:1000 in blocking agent PBS + 10% horse serum + 1 mM CaCl₂. After a 1-hr incubation at 37°C, the wells were washed and 100 µl of TMB was added to the well. After 2-3 min. at room temperature, the reaction was stopped by adding 100 µl H₂SO₄ 1.5 M. The absorbance at 450 nm was determined using a microplate reader. Normal mouse plasma did not interfere with the assay and the limit of sensitivity was 1 ng/ml for purified plasma-derived human FVIII added to normal mouse plasma.

### 3. Results

Human BM stromal cells transduced with the GALV-env pseudotyped PG13-F8 retroviral vector yielded an in vitro production of 490 ± 40 ng FVIII ng/10⁶ cells per 24 hr. One to 3x10⁶ transduced BM stromal cells were injected intrasplenically into NOD-SCID mice and FVIII expression was measured with a human FVIII-specific ELISA (Fig. 7C). Therapeutic levels of FVIII could be detected that were at least 10-fold higher than the therapeutic levels needed to convert severe to moderate hemophilia A (2 ng/ml). Therapeutic levels persisted for at least one to two weeks after which animals were sacrificed for PCR analysis to determine the homing pattern of the injected cells. Control animals that received PBS only did not produce human FVIII, as expected.

### 4. Discussion

Therapeutic human FVIII expression levels were detected in vivo in the plasma of these mice well above the levels needed to convert a severe to a moderate hemophilia A patient. This is the first demonstration that transduced BM stromal cells can be used to achieve therapeutic levels of a protein in vivo. More in particular, this represents the first protocol showing therapeutic levels of FVIII in vivo using BM stromal cells. Engraftment of the FVIII-engineered cells was relatively efficient since only a single injection of 1-3x10⁶ cells was needed to obtain FVIII expression. This is also the first demonstration that neo-organs or a conditioning regimen such as myelo-ablation are not required for BM stroma engraftment to achieve these therapeutic levels making it a clinically acceptable modality for hemophilia A gene therapy.

## Claims

1. Method for the ex vivo transduction of mammalian cells by means of a transduction protocol, which method comprises the steps of:
a) providing an intron-based retroviral vector comprising a B-domain deleted human factor VIII cDNA (designated as MFG-FVIIIΔB);
b) pseudotyping the said vector with the Gibbon ape leukemia virus (GALV) envelope;
c) transducing bone marrow stromal cells with the said pseudotyped vector by pre-incubating the cells for a suitable period of time in cell culture medium without phosphate and subsequently adding a vector-containing supernatant, optionally supplemented with transduction additives to the cells, followed by centrifuging the mixture thus obtained; and
d) optionally repeating step c).

2. Method as claimed in claim 1, wherein the mammalian cells are bone marrow stromal cells.

3. Method as claimed in claim 1, wherein the mammalian cells are bone marrow stromal precursor cells.

4. Method for the ex vivo transduction of mammalian cells by means of a transduction protocol, which method comprises the following elements:
a) the mammalian cell is transduced with a gene of interest;
b) the transduction is effected by means of a retroviral vector;
c) the vector comprises an intron;
d) the transduction protocol comprises pseudotyping of the vector;
e) the transduction protocol comprises a centrifugation step;
f) the transduction protocol comprises phosphate starvation of the cells to be transduced, wherein steps c) to f) are optional and can occur in any possible combination.

5. Method as claimed in claim 2, comprising the steps of:
a) providing an intron-based retroviral vector comprising any gene, in particular a gene that is secretable in the blood stream, more in particular a blood coagulation gene;
b) pseudotyping the said vector with the Gibbon ape leukemia virus (GALV) envelope;
c) transducing bone marrow stromal cells with the said pseudotyped vector by optionally pre-incubating the cells for a suitable period of time in cell culture medium without phosphate and subsequently adding a vector solution, optionally supplemented with transduction additives to the cells, followed by centrifugation of the mixture thus obtained; and
d) optionally repeating step c).

6. Method as claimed in claim 2, comprising the steps of:
a) providing an intron-based retroviral vector comprising any gene, in particular a gene that is secretable in the blood stream, more in particular a blood coagulation gene;
b) pseudotyping the said vector with the Gibbon ape leukemia virus (GALV) envelope;
c) transducing cells to be transduced, in particular bone marrow stromal precursor cells, with the said pseudotyped vector by optionally pre-incubating the cells for a suitable period of time in cell culture medium without phosphate and subsequently adding a vector solution, optionally supplemented with transduction additives to the cells, followed by centrifugation of the mixture thus obtained; and
d) optionally repeating step c).

7. Method as claimed in claim 2 for the ex vivo transduction of bone marrow stromal cells, comprising the steps of:
a) providing an intron-based retroviral vector comprising a B-domain deleted human factor VIII cDNA (designated as MFG-FVIIIΔB);
b) optionally pseudotyping the said vector with the Gibbon ape leukemia virus (GALV) envelope; and/or
c) optionally transducing bone marrow stromal cells with the said pseudotyped vector by pre-incubating the cells for a suitable period of time in cell culture medium without phosphate and subsequently adding a vector-containing solution, optionally supplemented with transduction additives to the cell; and/or
d) optionally centrifuging the mixture thus obtained; and/or
e) optionally repeating step c) and d).

8. Method as claimed in claim 2 for the ex vivo transduction of bone marrow stromal cells, comprising the steps of:
a) providing a retroviral vector comprising a B-domain deleted human factor VIII cDNA;
b) optionally pseudotyping the said vector with the Gibbon ape leukemia virus (GALV) envelope; and/or
c) optionally transducing bone marrow stromal cells with the said pseudotyped vector by pre-incubating the cells for a suitable period of time in cell culture medium without phosphate and subsequently adding a vector-containing solution, optionally supplemented with transduction additives to the cell; and/or
d) optionally centrifuging the mixture thus obtained; and/or
e) optionally repeating step c) and d).

9. Method as claimed in claim 2 for the ex vivo transduction of bone marrow stromal cells, comprising the steps of:
a) providing an intron-based retroviral vector comprising a gene of interest;
b) optionally pseudotyping the said vector with the Gibbon ape leukemia virus (GALV) envelope; and/or
c) optionally transducing bone marrow stromal cells with the said pseudotyped vector by pre-incubating the cells for a suitable period of time in cell culture medium without phosphate and subsequently adding a vector-containing solution, optionally supplemented with transduction additives to the cell; and/or
d) optionally centrifuging the mixture thus obtained; and/or
e) optionally repeating step c) and d).

10. Method as claimed in claim 2 for the ex vivo transduction of bone marrow stromal cells, comprising the steps of:
a) providing a retroviral vector comprising a gene of interest;
b) optionally pseudotyping the said vector with the Gibbon ape leukemia virus (GALV) envelope; and/or
c) optionally transducing bone marrow stromal cells with the said pseudotyped vector by pre-incubating the cells for a suitable period of time in cell culture medium without phosphate and subsequently adding a vector-containing solution, optionally supplemented with transduction additives to the cell; and/or
d) optionally centrifuging the mixture thus obtained; and/or
e) optionally repeating step c) and d).

11. Method as claimed in any one of the claims 7-10, wherein the gene of interest encodes a protein that is secretable in the blood stream.

12. Method as claimed in any one of the claims 2-10, wherein the gene of interest is a blood coagulation gene.

13. Method as claimed in claim 12, wherein the blood coagulation gene is selected from the group consisting of genes encoding factor VIII, factor IX, factor X, factor V, factor VII, factor XII, factor XIII, von Willebrand factor (vWF), tissue factor (TF).

14. Method as claimed in claims 4, 9 and 10, wherein the gene of interest encodes a protein that affects bone function, in particular bone marrow function and more in particular bone marrow stromal function.

15. Method as claimed in any one of the claims 1-6, wherein the mammalian cell is a bone marrow cell selected from the group consisting of bone marrow stromal stem cells, bone marrow stromal stem cells, bone marrow stromal precursor cells, bone marrow mesenchymal cells, bone marrow hematopoietic stem cells, bone marrow hematopoietic progenitor cells, cells belonging to the lymphohematopoietic lineage, fibroblasts, endothelial cells, chondroblasts, chondrocytes, myoblasts, myocytes, osteoblasts, epithelial cells.

16. Method as claimed in any one of the claims 1-6, wherein the mammalian cell is a cell selected from the group consisting of mesenthelial cells, keratinocytes, hepatocytes.

17. Method as claimed in any one of the claims 1-16, wherein instead of the GALV pseudotyping, the vector is pseudotyped with other envelopes that utilize the GLVR-1 receptor for viral entry, in particular the 10A1 envelope.

18. Method as claimed in any one of the claims 1-17, wherein the retroviral vector is a vector selected from the group consisting of Moloney murine leukemia virus, Gibbon ape leukemia virus, Rous sarcoma virus, myeloproliferative sarcoma virus, lentivirus, human foamy virus, human immunodeficiency virus (HIV), simian immunodeficiency virus (SIV), bovine leukemia virus (BLV).

19. Genetically engineered mammalian cells obtainable by performing the method as claimed in claims 1-18.

20. Genetically engineered bone marrow stromal cells obtained by performing the method as claimed in claims 1-3, 5-15, 17 and 18.

21. Cells as claimed in claims 19 or 20 for use in the therapeutical treatment of coagulation disorders, in particular hemophilia, more in particular hemophilia A.

22. Cells as claimed in claims 19 or 20 for use in the therapeutical treatment of bone marrow disorders, including osteoporosis, osteogenesis imperfecta, chondrodysplasia, arthritis and cancer.

23. Use of the cells as claimed in claims 19-21 for the preparation of a therapeutical composition for the treatment by means of gene therapy of disorders of the blood, in particular coagulation disorders, more in particular hemophilia A.

24. Use as claimed in claim 23, wherein the treatment for therapy of hemophilia A consists of returning bone marrow stromal cells, that have been genetically engineered with the gene for factor VIII by the method of claims 1-3, 5-15, 17 and 18 to a subject in need of therapy.

25. Use as claimed in claim 24, wherein the method of treatment comprises injection of 10⁸ autologous BM stromal cells prepared with the method of claims 1-3, 5-15, 17 and 18 to a subject to convert a severe to a mild hemophiliac.

26. Use as claimed in claim 24, wherein the method of treatment comprises injection of 7x10⁸ autologous BM stromal cells prepared with the method of claims 1-3, 5-15, 17 and 18 to a subject to completely correct the bleeding phenotype.

27. Use as claimed in claims 23-26, wherein the cells are returned to a patient in need of therapy in the absence of irradiation or other myeloablative regimens.


SEQUENCE LISTING

(1) GENERAL INFORMATION:

(i) APPLICANT:
(A) NAME: Leuven Research & Development vzw
(B) STREET: Groot Begijnhof/Benedenstraat 60
(C) CITY: Leuven
(E) COUNTRY: Belgium
(F) POSTAL CODE (ZIP): B-3000

(ii) TITLE OF INVENTION: Mammalian cell transduction for use in
gene therapy

(iii) NUMBER OF SEQUENCES: 8

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98.200382.4

(2) INFORMATION FOR SEQ ID NO: 1:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "primer"

(xi) SEQUENCE DESCRIPTION: SEQ ID NO: 1:

GCAGTTTTCT GTGCCCTTAT CGTC 24

(2) INFORMATION FOR SEQ ID NO: 2:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "primer"

(xi) SEQUENCE DESCRIPTION: SEQ ID NO: 2:

GGAGTTTATT TGGTTGCTGA CGG 23

1

(2) INFORMATION FOR SEQ ID NO: 3:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "primer"

(xi) SEQUENCE DESCRIPTION: SEQ ID NO: 3:

TTCAGGAAGC AGAGTCCCCA GT 22

(2) INFORMATION FOR SEQ ID NO: 4:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "primer"

(xi) SEQUENCE DESCRIPTION: SEQ ID NO: 4:

TGTCGATGTG GATTTTGCAG 20

(2) INFORMATION FOR SEQ ID NO: 5:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "primer"

(xi) SEQUENCE DESCRIPTION: SEQ ID NO: 5:

GAGCTCTCCA CCTGCTTCTT TCTG 24

(2) INFORMATION FOR SEQ ID NO: 6:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "primer"

(xi) SEQUENCE DESCRIPTION: SEQ ID NO: 6:

CCCTTCTCTA CATACTAGTA GGGC 24

(2) INFORMATION FOR SEQ ID NO: 7:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "primer"

(xi) SEQUENCE DESCRIPTION: SEQ ID NO: 7:

CATCTCCTGC TCGAAGTCTA GAGC 24

(2) INFORMATION FOR SEQ ID NO: 8:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "primer"

(xi) SEQUENCE DESCRIPTION: SEQ ID NO: 8:

CATTGTGATG GACTCCGGAG ACGG 24